# EUROPÄISCHE PATENTSCHRIFT

(11) **EP 2 120 906 B1**
(45) Veröffentlichungstag und Bekanntmachung des Hinweises auf die Patenterteilung: **22.04.2015**
(21) Anmeldenummer: 07857069.4
(22) Anmeldetag: 21.12.2007
(51) Int. Cl.: A61K 31/121, A61K 31/122, A61K 31/353, A61K 36/185, A61P 1/16, A61P 31/14, A61P 35/00

(54) **VERWENDUNG VON XANTHOHUMOL ALS WIRKSTOFF ZUR VORBEUGUNG UND/ODER BEKÄMPFUNG VON LEBERERKRANKUNGEN**
USE OF XANTHOHUMOL AS AN AGENT FOR PREVENTING AND/OR COMBATING LIVER DISEASES
UTILISATION DE XANTHOHUMOL EN TANT QUE PRINCIPE ACTIF POUR PRÉVENIR ET/OU LUTTER CONTRE LES PATHOLOGIES DU FOIE

(30) Priorität: 22.12.2006 DE 102006062264
(43) Veröffentlichungstag der Anmeldung: 25.11.2009
(73) Patentinhaber: Flaxan GmbH & Co. KG, 90482 Nürnberg (DE)
(72) Erfinder: HELLERBRAND, Claus, 93053 Regensburg (DE)
(74) Vertreter: Stippl, Hubert
(86) Internationale Anmeldenummer: PCT/EP2007/011358
(87) Internationale Veröffentlichungsnummer: WO 2008/077618

(56) Entgegenhaltungen:
- EP-A- 1 543 834
- EP-A1- 1 277 473
- WO-A-01/03681
- WO-A-2007/016578
- US-A1- 2006 276 372
- LEE TZONG-HSIEN ET AL: "Enhanced nuclear factor-kappa B-associated Wnt-1 expression in hepatitis B- and C-related hepatocarcinogenesis: identification by functional proteomics" JOURNAL OF BIOMEDICAL SCIENCE, Bd. 13, Nr. 1, Januar 2006 (2006-01), Seiten 27-39, XP002473939 ISSN: 1021-7770
- ALBINI ADRIANA ET AL: "Mechanisms of the antiangiogenic activity by the hop flavonoid xanthohumol: NF-kappa B and Akt as targets" FASEB JOURNAL, Bd. 19, Nr. 14, Dezember 2005 (2005-12), XP002473940 ISSN: 0892-6638
- XINGJUN LIU ET AL: "Smad3 Specific Inhibitor, Naringenin, Decreases the Expression of Extracellular Matrix Induced by TGF-[beta]1 in Cultured Rat Hepatic Stellate Cells" PHARMACEUTICAL RESEARCH, KLUWER ACADEMIC PUBLISHERS-PLENUM PUBLISHERS, NE, Bd. 23, Nr. 1, 1. Januar 2006 (2006-01-01), Seiten 82-89, XP019370960 ISSN: 1573-904X
- BUCKWOLD V E ET AL: "Antiviral activity of hop constituents against a series of DNA and RNA viruses" ANTIVIRAL RESEARCH, ELSEVIER SCIENCE BV., AMSTERDAM, NL, Bd. 61, Nr. 1, Januar 2004 (2004-01), Seiten 57-62, XP002398448 ISSN: 0166-3542
- DIETZ BIRGIT M ET AL: "Xanthohumol isolated from Humulus lupulus inhibits menadione-induced DNA damage through induction of quinone reductase" CHEMICAL RESEARCH IN TOXICOLOGY, Bd. 18, Nr. 8, August 2005 (2005-08), Seiten 1296-1305, XP002473941 ISSN: 0893-228X
- GERHAUSER CLARISSA ET AL: "Cancer chemopreventive activity of Xanthohumol, a natural product derived from hop" MOLECULAR CANCER THERAPEUTICS, Bd. 1, Nr. 11, September 2002 (2002-09), Seiten 959-969, XP002473942 ISSN: 1535-7163
- NIKOLIC D ET AL: "Metabolism of xanthohumol and isoxanthohumol, prenylated flavonoids from hops (Humulus lupulus L.), by human liver microsomes" JOURNAL OF MASS SPECTROMETRY, WILEY, CHICHESTER, GB, Bd. 40, Nr. 3, 15. Februar 2005 (2005-02-15), Seiten 289-299, XP002393640 ISSN: 1076-5174
- BOSSERHOFF ANJA ET AL: "Obesity and Fatty Liver Are 'Grease' for the Machinery of Hepatic Fibrosis", DIGESTIVE DISEASES, vol. 29, no. 4, 2011, pages 377-383, ISSN: 0257-2753(print)
- ROMBOUTS KRISTA ET AL: "Molecular Mechanisms of Hepatic Fibrosis in Non-Alcoholic Steatohepatitis", DIGESTIVE DISEASES, vol. 28, no. 1, 2010, pages 229-235, ISSN: 0257-2753

## Beschreibung

Die vorliegende Erfindung betrifft die Verwendung von Xanthohumol als Wirkstoff für die Herstellung eines Nahrungsergänzungsmittels oder Medikaments zur Vorbeugung von vermittelten Leberschädigungen.

### Stand der Technik

Xanthohumol ist ein Prenylflavenoid, welches im Hopfen vorkommt. Verschiedene Untersuchungen zeigen biologische Effekte von Xanthohumol.

So wird beispielsweise in der EP 1 543 834 A 1 die anti-karzenogene Wirkung von Xanthohumol beschrieben.

Aus der EP 0 679 393 B 1 ist bekannt, dass Xanthohumol eine stark inhibierende Wirkung gegenüber einer Knochenresorption aufweist und daher als Mittel zur Behandlung von Osteoporose eingesetzt werden kann.

Die DE 103 08 864 A 1 beschreibt ein neues Brauverfahren, mit dem ein Bier hergestellt werden soll, welches aufgrund eines besonderen Brauverfahrens einen erhöhten Anteil an Xanthohumol und damit gesteigerte gesundheitsfördernde Wirkungen begründet.

Die EP 1 543 834 A1 betrifft die Herstellung von Hopfenextrakten mit östrogener und antiproliferativer Bioaktivität. Hierbei wird die proöstrogene Wirkung von Isoxanthohumol als Isomerisationsprodukt von Xanthohumol genutzt, um ein Arzneimittel zur Chemoprävention von Krebserkrankungen bereitzustellen.

WO 01/03681 A2 beschreibt verschiedene Wirksamkeiten von Naringenin.

Die US 2006/0276372 A1 betrifft die Verwendung von Carotinoid-Xanthohumol Konjugaten zur Behandlung von Leberkrebs.

In LEE TZONG-HSIEN ET AL: "Enhanced nuclear factor-kappa B-associated Wnt-1 expression in hepatitis B- and C-related hepatocarcinogenesis: identification by functional proteomics" JOURNAL OF BIOMEDICAL SCIENCE, Bd. 13, Nr. 1, Januar 2006 (2006-01), Seiten 27-39, XP002473939 ISSN: 1021-7770 wird der NF-Kappa B Faktor als Ansatzpunkt für die Behandlung des hepatozellulären Karzinoms, welches durch NF-Kappa B Aktivierung infolge von HBV oder HCV und Zirrhose induziert wird, vorgeschlagen.

In ALBINI ADRIANA ET AL: "Mechanisms of the antiangiogenic activity by the hop flavonoid xanthohumol: NF-kappa B and Akt as targets" FASEB JOURNAL, Bd. 19, Nr. 14, Dezember 2005 (2005-12), XP002473940 ISSN: 0892-6638 wird darauf abgestellt, dass Xanthohumol den Signalübertragungsweg hemmt und damit das Wachstum von Gefäßkrebszellen unterbindet.

XINGJUN LIU ET AL: "Smad3 Specific inhibitor, Naringenin, Decreases the Expression of Extracellular Matrix Induced by TGF-[β]1 in Cultured Rat Hepatic Stellate Cells" PHARMACEUTICAL RESEARCH, KLUWER ACADEMIC PUBLISHERS-PLENUM PUBLISHERS, NE, Bd. 23, Nr. 1, 1. Januar 2006 (2006-01-01), Seiten 82-89, XP019370960 ISSN: 1573-904X behandelt den Einsatz von Naringenin und beschreibt in diesem Zusammenhang eine antifibrogene Wirkung.

BUCKWOLD V E ET AL: "Antiviral activity of hop constituents against a series of DNA and RNA viruses" ANTIVIRAL RESEARCH, ELSEVIER SCIENCE BV., AMSTERDAM, NL, Bd. 61, Nr. 1, Januar 2004 (2004-01), Seiten 57-62, XP002398448 ISSN: 0166-3542 betrifft den Einsatz von Xanthohumol zur Bekämpfung unterschiedlicher Viruserkrankungen.

DIETZ BIRGIT M ET AL: "Xanthohumol isolated from Humulus lupulus inhibits menadione-induced DNA damage through induction of quinone reductase" CHEMICAL RESEARCH IN TOXICOLOGY, Bd. 18, Nr. 8, August 2005 (2005-08), Seiten 1296-1305, XP002473941 ISSN: 0893-228X beschreibt den Einfluss von Xanthohumol auf toxisch geschädigte Leberkrebszellen.

GERHAUSER CLARISSA ET AL: "Cancer chemopreventive activity of Xanthohumol, a natural product derived from hop" MOLECULAR CANCER THE-RAPEUTICS, Bd. 1, Nr. 11, September 2002 (2002-09), Seiten 959-969, XP002473942 ISSN: 1535-7163 beschreibt den wachstumshemmenden Einfluss von Xanthohumol zur Hemmung der Vermehrung von Krebszellen allgemein.

NIKOLIC D ET AL: "Metabolism of xanthohumol and isoxanthohumol, prenylated flavonoids from hops (Humulus lupulus L.), by human liver microsomes" JOURNAL OF MASS SPECTROMTETRY, WILEY, CHICHESTER, GB, Bd. 40, Nr. 3, 15 Februar 2005 (2005-02-15). Seiten 289-299, XP002393640 ISSN: 1076-5174 offenbart Vorgänge des Stoffwechsels bei der Einnahme von Xanthohumol und Isoxanthohumol und verweist auf eine Nützlichkeit des Xanthohumols als krebspräventiver Wirkstoff.

### Aufgabenstellung

Die vorliegende Erfindung hat sich zur Aufgabe gestellt, weitere gesundheitsfördernde Anwendungen von Xanthohumol aufzufinden.

Diese Aufgabe wird durch die Merkmale der Ansprüche 1 sowie 14 gelöst. Xanthohumol hat keinerlei Nebenwirkungen. Dies ermöglicht es, wirkungsvoll über eine lange Zeit chronischen Lebererkrankungen, insbesondere chronischen Lebererkrankungen durch regelmäßige Einnahme, prophylaktisch vorzubeugen. Es hat sich bei Untersuchungen überraschend herausgestellt, dass Xanthohumol Stoffwechselmechanismen hemmt, die von ganz besonderer Bedeutung für die durch Adipositas (Übergewicht) und Diabetes vermittelte Leberschädigung sind. Adipositas und Diabetes sind für einen großen Teil der Leberzirrhosen verantwortlich. Die Tendenz ist steigend. Insgesamt stellen chronische Lebererkrankungen mittlerweile ein bedeutendes wirtschaftliches Problem dar. Durch kontinuierliche Einnahme von Xanthohumol kann ein wirksamer prophylaktischer Schutz ohne Nebenwirkungen für die gesamte Bevölkerung aufgebaut werden.

Für die Behandlung der Leberfibrose gibt es derzeit noch keine gesicherten Therapieformen. Eine Inhibierung bzw. ein Stopp der Fibrosierung kann nur durch die Eliminierung der schädigenden Ursache, also z. B. im Fall von Hepatitis-VirusInfektion durch Eliminierung der Hepatitis-Viren erreicht werden. Die Eliminierung der schädigenden Ursache gelingt jedoch nur bei einem Teil der Patienten mit chronischen Lebererkrankungen bzw. ist bei Patienten mit genetischen Lebererkrankungen derzeit generell nicht möglich. Im Falle von Hepatitis-VirusInfektionen ist hier bisher ein Einsatz von Medikamenten mit starken Nebenwirkungen erforderlich. Selbst bei Einsatz solcher Medikamente wird lediglich bei einem Teil der Patienten eine Virus-Elimination erreicht. Der Einsatz von Xanthohumol kann hier erfreulicherweise Abhilfe schaffen.

Gemäß der Erfindung kann das Xanthohumol gerade bei Personen mit einem hohen Risikoprofil (Adipösen, Diabetiker) prophylaktisch eingesetzt werden.

Was die Darreichung anbelangt, ist gemäß der Erfindung eine Verwendung dahingehend vorgesehen, dass das Xanthohumol als wirksamer Bestandteil einer pharmazeutischen Zusammensetzung mit einem pharmazeutisch annehmbaren Träger wie z. B. Mannit, Saccharose, Laktose, Glukose, Fructose, Maltose usw. zugeführt werden.

Xanthohumol eignet sich ganz besonders dafür, als Wirkstoff einem Nahrungsmittel beigesetzt zu werden bzw. einem Getränk beigemischt zu werden.

Die Zugabe von Xanthohumol als Wirkstoff erfolgt insbesondere dazu, die Aktivität von freien Sauerstoffradikalen konkret in der Leber zu reduzieren oder zu unterbinden. Es wurde nämlich herausgefunden, dass bei einer Schädigung der Leber in jeglicher Form, d. h. entweder durch Entzündung (beispielsweise durch Viren, durch übermäßigen Alkoholgenuss, durch Fettleibigkeit und/oder Diabetes oder Strahlenbelastung) freie Sauerstoffradikale entstehen, die zur Entstehung einer Leberentzündung, Leberfibrose beitragen. Die Entstehung der freien Sauerstoffradikalen wird durch das Xanthohumol gehemmt bzw. deren Aktivität beeinträchtigt. Daraus ergibt sich der Vorteil, dass durch Xanthohumol alle drei vorgenannten Schädigungsmechanismen der Leber gleichermaßen wirksam beeinflusst werden können.

Insbesondere wurde herausgefunden, dass die Zugabe von Xanthohumol besonders dazu geeignet ist, den so genannten NF-Kappa-B-Faktor entscheidend zu beeinflussen, d. h. insbesondere dessen Aktivität zu reduzieren oder zu unterbinden. NF-Kappa-B ist ein Vermittler von Signalen in der Zelle und an der Modulation zahlreicher Zellfunktionen beteiligt. Es wurde gefunden, dass gerade der NF-Kappa-B-Faktor für die vorgenannten drei Schädigungsmechanismen der Leber eine ganz besondere Rolle spielt. Auch für die Entstehung und das Voranschreiten von NASH spielt NF-Kappa-B eine wichtige Rolle.

Des Weiteren wurde herausgefunden, dass das Xanthohumol in überraschender Weise in vergleichsweise hohen Dosierungen eingesetzt werden kann. Denn es hat sich herausgestellt, dass auch bei höheren Dosierungen keine die Zellen schädigende Wirkung von Xanthohumol auftritt, so dass eine selektive Wirksamkeit gegeben ist.

Es wurde herausgefunden, dass mit zunehmender Dosierung an Xanthohumol beispielsweise beginnend mit einer Untergrenze von 5 µM ein kontinuierlicher Anstieg der positiven Wirkung festgestellt werden kann, dies insbesondere bis zu einer Maximalgrenze von 100 µM. Daraus resultiert der Vorteil, dass je nach beabsichtigtem Einsatz des Behandlungsmittels (Nahrungsmittel mit Anteil an Xanthohumol zur täglichen Einnahme als Prophylaxe oder als Arzneimittel zur Therapie) Präparate mit unterschiedlichen Dosierungen zielgerichtet angeboten werden können.

Beispielsweise können bei einer chronischen Erkrankung mehrere Wirkungsmechanismen gleichzeitig auftreten, so dass durch das erfindungsgemäße Präparat die drei Wirkungsmechanismen der Leberentzündung, als auch der Wirkungsmechanismus der Leberzirrhose bzw. Leberfibrose als auch der Wirkungsmechanismus der Entstehung von Leberkrebs als auch der Wirkungsmechanismus des Voranschreitens des Leberkrebses maßgeblich gleichzeitig bekämpft werden können.

Zweckmäßigerweise ist der Wirkstoff, d. h. das Xanthohumol in einer Darreichungsform (Applikation und/oder Dosierung) anzuwenden, so dass sich in der Leber Wirkstoffkonzentrationen ≥ 5 µM, insbesondere ≥ 10 µM, insbesondere ≥ 20 µM, insbesondere ≥ 30 µM, insbesondere ≥ 40 µM, insbesondere ≥ 50 µM einstellen.

Vorzugsweise ist der Wirkstoff in einer Darreichungsform anzuwenden, dass sich in der Leber eine Wirkstoffkonzentration von maximal 100 µM einstellt.

Anwendungsfallbezogen ist der jeweilige Wirkstoff in einer Darreichungsform anzuwenden, so dass sich in der Leber Wirkstoffkonzentrationen in folgenden Bereichen einstellen, 1 bis 100 µM, vorzugsweise 1 - 25 µM, vorzugsweise 1 - 10 µM oder 5 - 100 µM, vorzugsweise 10 - 50 µM, vorzugsweise 10 - 25 µM. Je nach Anwendungsfall können die betreffenden Bereiche ausgewählt werden. Insbesondere genügen bei der Behandlung von Fibrose bereits vergleichsweise geringe Dosierungen, wohingegen bei der Behandlung von Leberkrebs erhöhte Dosierungen zweckmäßig sind.

Wird Xanthohumol beispielsweise der der Nahrung bzw. als Tablette verabreicht, kann somit aufgrund der Aufnahme über den Darm es zu vergleichsweise hohen Xanthohumol- Spiegeln kommen, die sich nach der Passage durch die Leber aber rasch verdünnen, d.h. in keinem anderen Organ auch nur ein annähernd ein so hoher Xanthohumol-Spiegel mehr erreicht wird.

Hinsichtlich der Gewinnung von Xanthohumol aus Hopfenpflanzen wird auf die EP 0 679 393 B 1 und die EP 1 543 834 A 1 vollinhaltlich verwiesen.

Gemäß der vorliegenden Erfindung kann eine Zusammensetzung als Wirksubstanz verwendet werden, bei der Xanthohumol nicht in Reinform, sondern als Hopfenextraktionsprodukt vorliegt. Es wurde festgestellt, dass neben dem Xanthohumol vorliegende Trägerbestandteile, die herstellungsbedingt vorliegen, die Aufnahme der Wirksubstanz in den Organismus und damit die Wirksamkeit sogar noch unterstützen können.

Zweckmäßigerweise ist die Dosis zur Verabreichung der Wirksubstanz bezogen auf den jeweiligen (Rein-)Anteil an Wirksubstanz größer als 0,01 mg / kg Körpergewicht / Tag, vorzugsweise größer als 0,1 mg / kg Körpergewicht / Tag, vorzugsweise größer als 1 mg / kg Körpergewicht / Tag, vorzugsweise größer als 10 mg / kg Körpergewicht / Tag, vorzugsweise größer als 50 mg / kg Körpergewicht / Tag, vorzugsweise größer als 100 mg / kg Körpergewicht / Tag, wobei es sich bei dem Körpergewicht um das Körpergewicht eines Menschen handelt.

Zweckmäßigerweise ist die Dosis zur Verabreichung bezogen auf den jeweiligen (Rein-)Anteil an Wirksubstanz kleiner als 161 mg / kg Körpergewicht / Tag, vorzugsweise kleiner als 50 mg / kg Körpergewicht / Tag, vorzugsweise kleiner als 10 mg / kg Körpergewicht / Tag, vorzugsweise kleiner als 1 mg / kg Körpergewicht / Tag, vorzugsweise kleiner als 0,1 mg / kg Körpergewicht / Tag, wobei es sich bei dem Körpergewicht um das Körpergewicht eines Menschen handelt.

Zweckmäßigerweise ist die Dosis zur Verabreichung bezogen auf den jeweiligen (Rein-)Anteil an Wirksubstanz ein Bereich von 0,01 bis 161 mg / kg Körpergewicht / Tag, vorzugsweise 0,05 bis 120 mg / kg Körpergewicht / Tag, vorzugsweise 0,1 bis 100 mg / kg Körpergewicht / Tag, vorzugsweise 0,5 bis 80 mg / kg Körpergewicht / Tag, vorzugsweise 1 bis 80 mg / kg Körpergewicht / Tag, vorzugsweise 5 bis 80 mg / kg Körpergewicht / Tag, vorzugsweise 10 bis 80 mg / kg Körpergewicht / Tag, wobei es sich bei dem Körpergewicht um das Körpergewicht eines Menschen handelt.

In vorteilhafter Weise liegen die Anteile des Xanthohumol im Bereich von 0,1 Gew.-% - 99 Gew.-%, vorzugsweise 5 Gew.-% - 99 Gew.-%, vorzugsweise 10 Gew.-% - 99 Gew.-%, vorzugsweise 20 Gew.-% - 99 Gew.-%, vorzugsweise 30 Gew.-% - 99 Gew.-%, vorzugsweise 40 Gew.-% - 99 Gew.-%, vorzugsweise 50 Gew.-% - 99 Gew.-%, vorzugsweise 60 Gew.-% - 99 Gew.-%, vorzugsweise 70 Gew.-% - 99 Gew.-%.

Sofern neben dem Xanthohumol als Wirkstoff weitere Bestandteile, insbesondere natürliche Bestandteile aufgrund der Gewinnung des Xanthohumols aus Hopfen vorhanden sind, kann dies die Wirksamkeit sogar noch erhöhen, da diese Bestandteile eine verbesserte Aufnahme des Wirkstoffes im Organismus bewirken.

Alternativ kann das Xanthohumol aber auch in Reinform verwendet werden.

Zudem ist gemäß der vorliegenden Erfindung auch die Verwendung von Xanthohumol in synthetisierter Form möglich.

Gemäß einer weiteren Ausgestaltung wird das Xanthohumol, in Verbindung mit mindestens einem weiteren Wirkstoff eingesetzt. Bei diesem Wirkstoff kann es sich vorzugsweise um einen Wirkstoff handeln, welcher die Verträglichkeit und/oder die Aufnahme des zu verabreichenden Wirkstoffes im Körper und/oder dessen Wirksamkeit und/oder dessen Stabilität und/oder dessen Handhabbarkeit positiv beeinflusst.

Das Xanthohumol bzw. ein Metabolit davon können in Verbindung mit oder auf der Basis von einem Alkali- oder Erdalkalisalz zum Einsatz kommen.

Das zu verabreichende Mittel kann insbesondere als Flüssigkeit, Brei, Emulsion, in Form von Nanopartikeln, als Puder oder als Gel zum Einsatz kommen. Die Darreichung kann als eigenständiges Medikament, aber auch als Zusatz zu einem flüssigen oder festen Lebensmittel, je nachdem, ob eine Therapie oder eine Prophylaxe gewünscht ist, erfolgen.

Der Wirkstoff kann unter Einsatz von pharmazeutisch gerechten, Nährstoff- gerechten oder lebensmittelgerechten Lösungsmitteln, Trägerstoffen oder Additiven wie z. B. Stärke, Dextrin, insbesondere Zyklodextrin, Maltodextrin, Proteinen, Methylzellulose, Carbomethoxyzellulose oder Xanthan erfolgen.

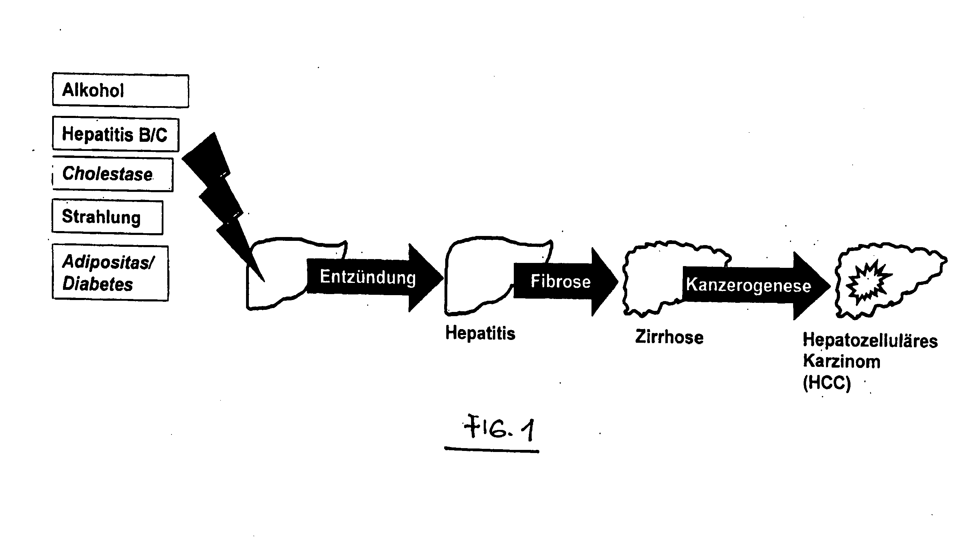

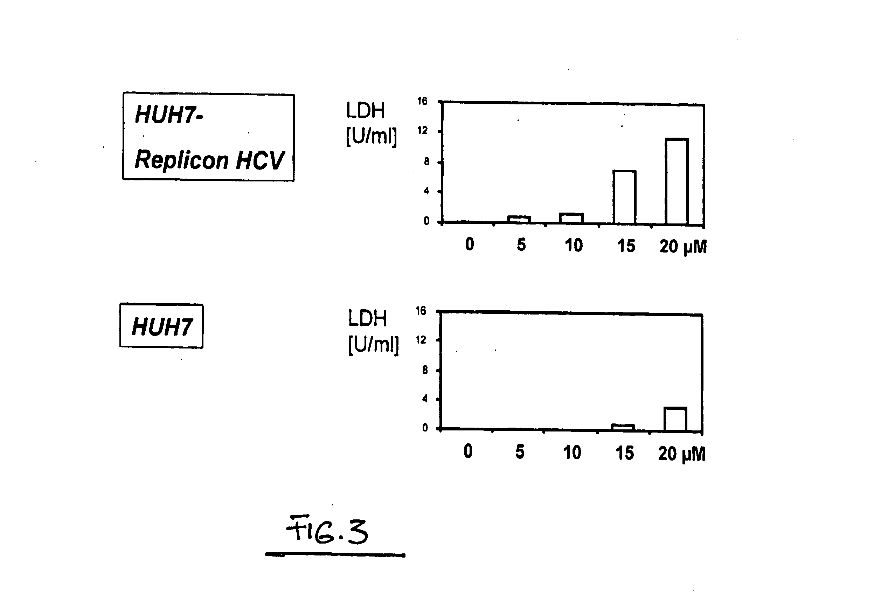

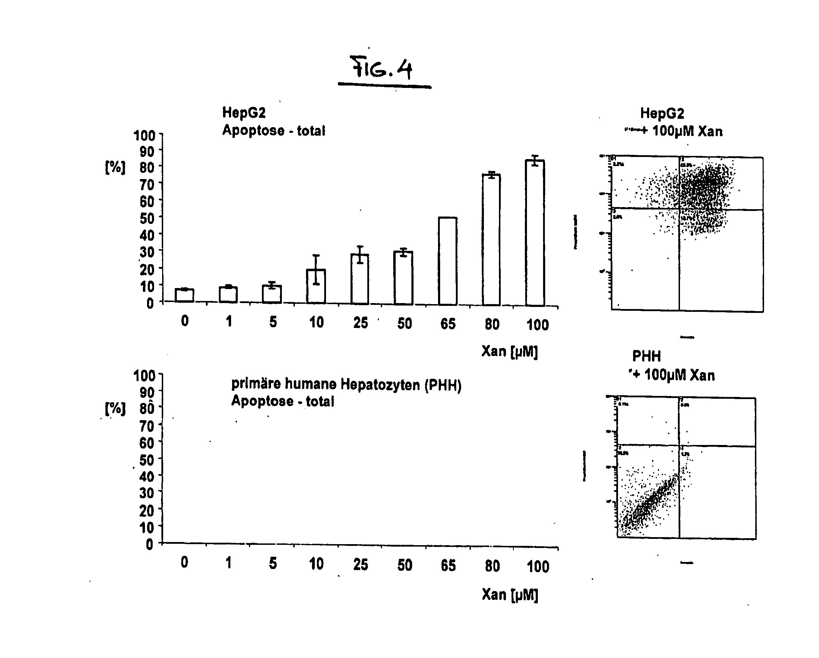

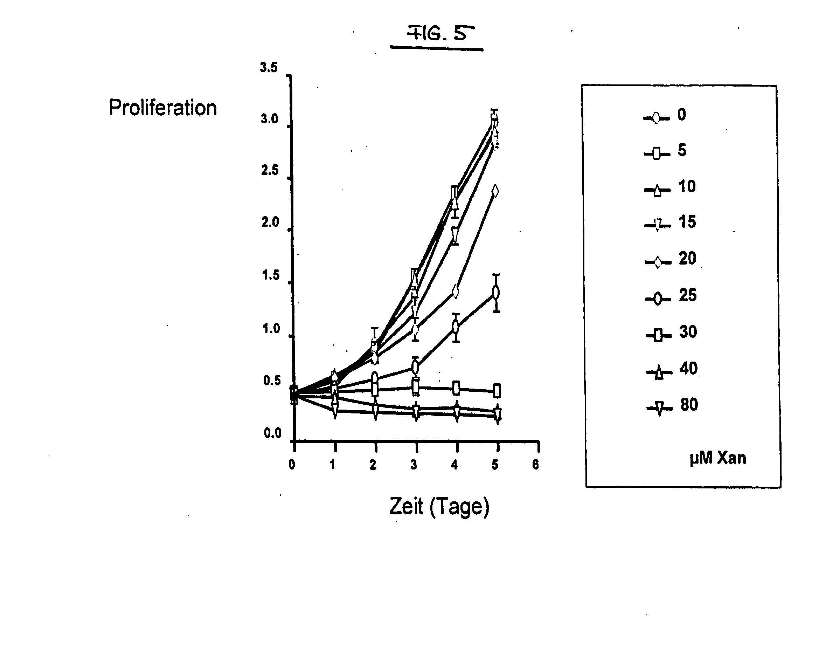

Die Untersuchungen gemäß den nachfolgenden Figuren 1 - 9 wurden mit Xanthohumol in Reinform (> 98%) durchgeführt.

### Beispiel 1

Nachstehend wird eine beispielhafte Zusammensetzung eines Arzneimittels wiedergegeben.

### Pulvermischung zur Direktverpressung

| | |
|---|---|
| Xanthohumol (Reinsubstanz) | 5 g |
| Mikrokristalline Zellulose | 10 Gew. % |
| Natriumcarboxymethylstärke | 3 Gew. % |
| Hochdisperses Siliciumdioxid | 1 Gew. % |
| Magnesiumstearat | 1 Gew. % |
| Tablettose (Lactose-Monohydrat) | Rest auf 100 Gew. % |

### Beispiel 2

Nachstehend wir eine beispielhafte Zusammensetzung eines Nahrungsmittels mit hinzugefügtem Xanthohumol als Wirkstoff wiedergegeben.

### Xanthohumol (Reinsubstanz in Pulverform) 500 mg pro 200 ml Milchprodukt (cremig, z. B. Joghurt)

Die obige Zusammensetzung für ein Nahrungsmittel erlaubt aufgrund der einfach durchzuführenden Beimengung in ein cremiges Nahrungsmittel eine für die erforderliche Menge von Xanthohumol optimale Darreichung.

Figur 1 zeigt die therapeutischen Zielsetzungen von Xanthohumol. Die Darstellung spiegelt die Kette der Wirkungsmechanismen ausgehend von einer Lebererkrankung bedingt beispielsweise durch Alkohol, Viren, Strahlen, Adipositas und/oder Diabetes bis hin zum Leberkrebs wider. Die Verwendung eines Präparats mit Xanthohumol beeinträchtigt in vorteilhafter Weise sämtliche Stufen der Wirkungskette gemäß Figur 1. Xanthohumol kann aber auch gezielt bei der Behandlung einzelner Stationen der Wirkungsstellen erfolgreich eingesetzt werden.

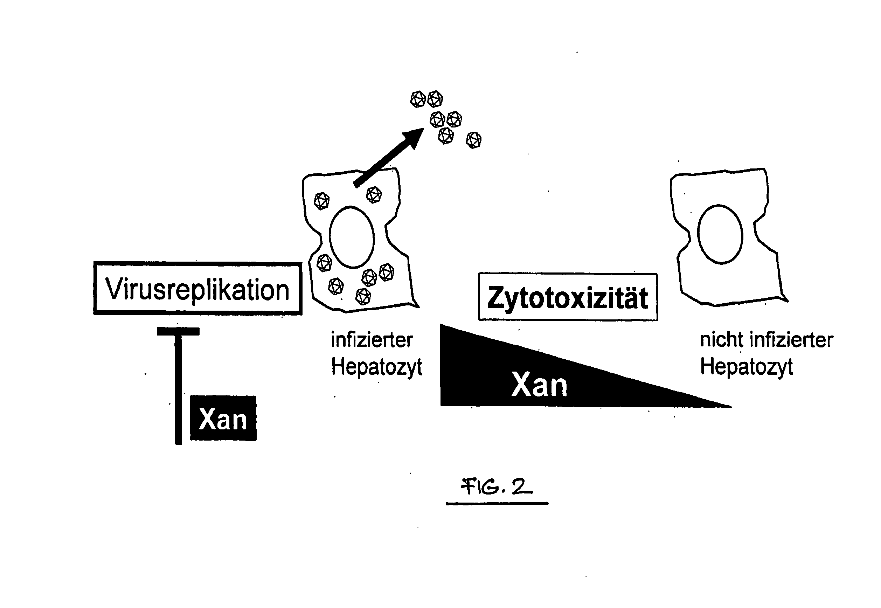

Figur 2 zeigt eine schematische Darstellung der Wirksamkeit von Xanthohumol bei einer viralen Schädigung der Leber, insbesondere durch Hepatitis B und C. Es wurde herausgefunden, dass die vorgenannten Wirkstoffe nicht nur die Virusvermehrung in vorteilhafter Weise hemmen, sondern darüber hinaus auch eine selektive Abtötung der bereits vom Virus betroffenen körpereigenen Leberzellen gewährleistet, wohingegen gesunde Leberzellen unbeeinträchtigt bleiben. Durch die Erfindung wird demzufolge eine gezielte Therapie zur Reduzierung bzw. Beseitigung von mit dem Virus infizierten Leberzellen ermöglicht.

Figur 3 zeigt anhand einer Diagrammgegenüberstellung die selektive Wirksamkeit des Einsatzes von Xanthohumol in Bezug auf mit Hepatitis C infizierten Leberzellen sowie nicht mit Hepatitis C infizierten Leberzellen im Vergleich.

Figur 4 zeigt eine grafische Darstellung der Wirksamkeit des Einsatzes von Xanthohumol in Bezug auf Apoptose (programmierter Zelltod) von Leberkrebszellen (HepG2) im Vergleich zu gesunden Leberzellen (primäre humane Hepatozyten).

Figur 5 zeigt eine Gegenüberstellung des Wachstums von Leberkrebszellen (HepG2) über der Zeit in Abhängigkeit der Dosierung von Xanthohumol. Wie aus der Darstellung deutlich wird, wird das Wachstum der Krebszellen mit zunehmender Konzentration an Xanthohumol fortschreitend gehemmt.

Die Figuren 6 und 7 veranschaulichen den Effekt der Zugabe von Xanthohumol in Bezug auf die Prävention der Transformation körpereigener Leberzellen in hepatische Sternzellen, welche für die Vernarbung der Leber bei Leberzirrhose verantwortlich sind.

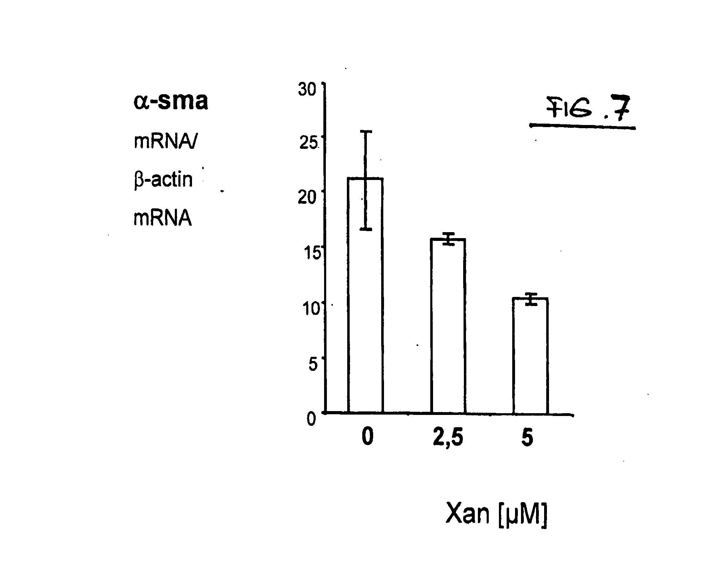

Wie aus Figur 7 ersichtlich ist, wird mit zunehmender Dosierung von Xanthohumol die Bildung von Narbengewebe zunehmend unterdrückt.

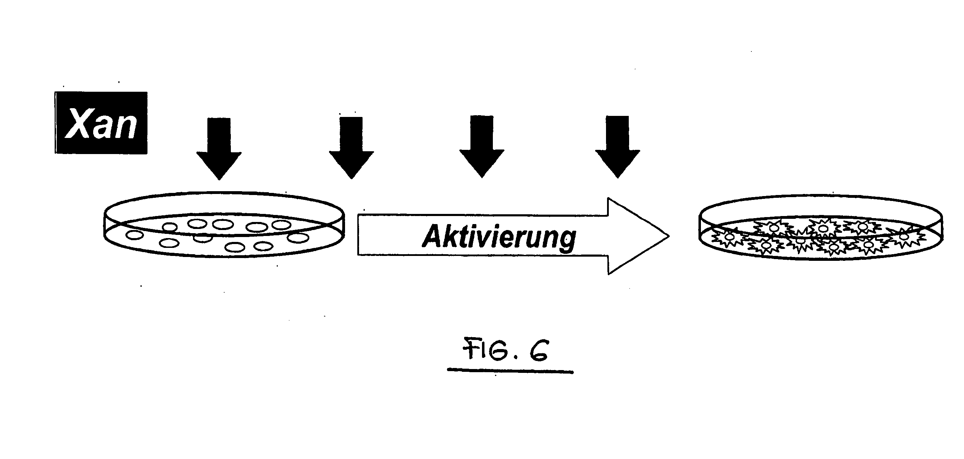

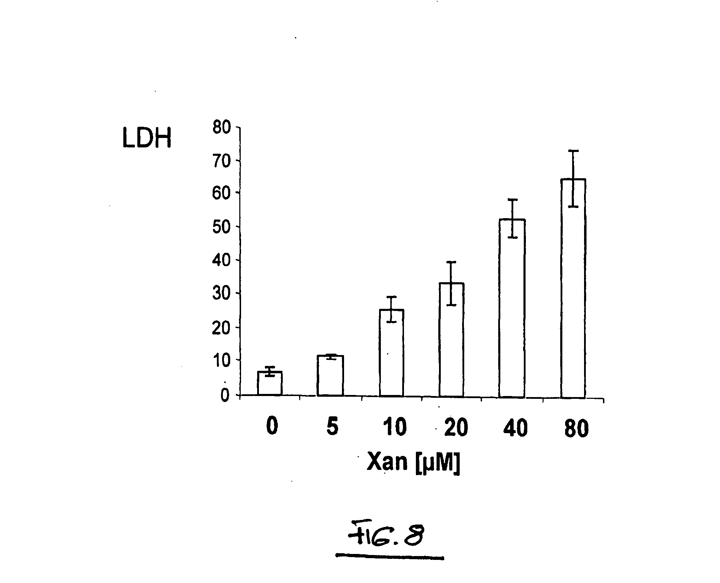

Aus Figur 8 ist eine Darstellung der Wirkung von Xanthohumol in zunehmender Dosierung auf die bereits vorhandenen, aktivierten hepatischen Sternzellen ersichtlich. Aus der Darstellung gemäß Figur 6 ist ersichtlich, dass mit zunehmender Dosierung an Xanthohumol ein erhöhter Effekt der Abtötung (LDH) aktivierter, hepatischer Sternzellen sich einstellt.

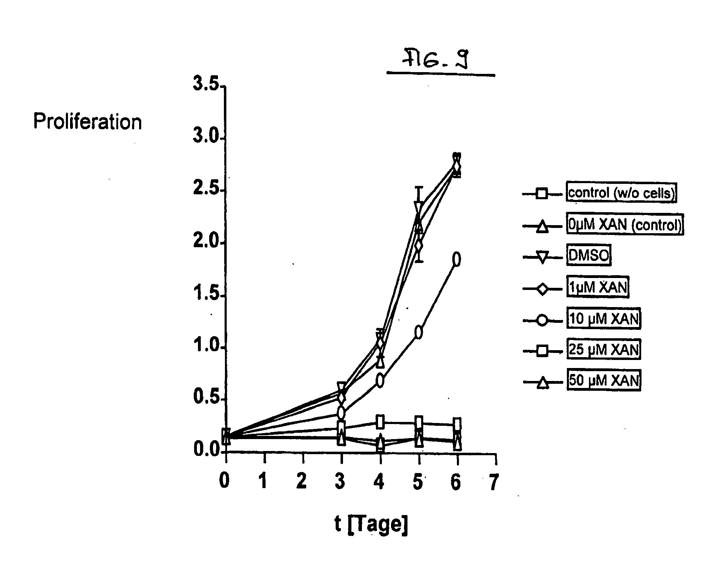

Aus Figur 9 ergibt sich der Einfluss der Dosierung von Xanthohumol auf das Wachstum der aktivierten hepatischen Stemzellen.

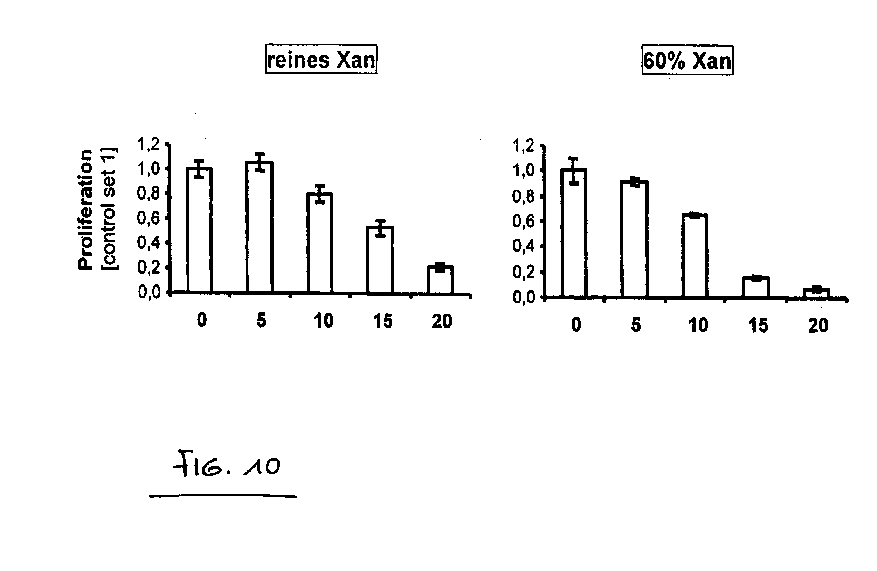

Figur 10 zeigt eine Gegenüberstellung der Lebensdauer (Proliferation) von Leberkrebszellen nach Anwendung von Xanthohumol in Reinform (> 98%) bzw. in einer Form, bei der Xanthohumol als Anteil zu 60% vorliegt. Bei Letzterem handelt es sich um das aus Hopfenextrakt in einem üblichen technischen Prozess gewonnene Xanthohumol, welches weitere, natürliche Bestandteile enthält. Je niedriger die Balken sind, umso mehr Zellen werden in ihrem Wachstum gehemmt.

Es zeigt sich, dass sich bei 60%-igem Xanthohumol sogar noch eine stärkere Wirkung einstellt als bei Xanthohumol in Reinform. Dies ist darauf zurückzuführen, dass die restlichen Bestandteile bei dem natürlichen Xanthohumol eine Trägerfunktion haben und damit eine wirkungsvollere Zuführung des Wirkstoffs in den Organismus begründen.

## Patentansprüche

1. Verwendung von Xanthohumol mit der Formel 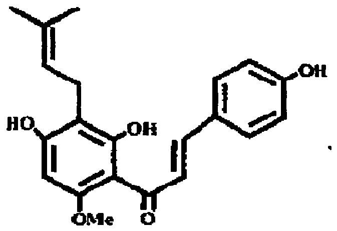 gegebenenfalls in Verbindung mit oder auf der Basis von einem Salz als Wirkstoff für die Herstellung eines Nahrungsergänzungsmittels oder Medikaments zur Vorbeugung durch Adipositas und/oder Diabetes vermittelten Leberschädigungen.

2. Verwendung nach Anspruch 1,
**dadurch gekennzeichnet, dass**
der jeweilige Wirkstoff in einer Darreichungsform anzuwenden ist, so dass sich in der Leber Wirkstoffkonzentrationen in einer Dosierung von ≥ 5 µM, insbesondere ≥ 10 µM, insbesondere ≥ 20 µM, insbesondere ≥ 30 µM, insbesondere ≥ 40 µM, insbesondere ≥ 50 µM einstellen.

3. Verwendung nach Anspruch 1 oder 2,
**dadurch gekennzeichnet, dass**
der Wirkstoff in einer Darreichungsform anzuwenden ist, so dass sich in der Leber eine Wirkstoffkonzentration von maximal 100 µM einstellt.

4. Verwendung nach Anspruch 1,
**dadurch gekennzeichnet, dass**
das Xanthohumol in einer Darreichungsform anzuwenden ist, so dass sich in der Leber Wirkstoffkonzentrationen in folgenden Bereichen einstellen, 1 bis 100 µM, vorzugsweise 1 - 25 µM, vorzugsweise 1 - 10 µM oder 5 - 100 µM, vorzugsweise 10 - 50 µM, vorzugsweise 10 - 25 µM einstellen.

5. Verwendung nach Anspruch 1,
**dadurch gekennzeichnet, dass**
die Dosis bezogen auf den Anteil an Wirksubstanz größer ist als 0,01 mg / kg Körpergewicht / Tag, vorzugsweise größer ist als 0,1 mg / kg Körpergewicht / Tag, vorzugsweise größer ist als 1 mg / kg Körpergewicht / Tag, vorzugsweise größer ist als 10 mg / kg Körpergewicht / Tag, vorzugsweise größer ist als 50 mg / kg Körpergewicht / Tag, vorzugsweise größer ist als 100 mg / kg Körpergewicht / Tag, wobei es sich bei dem Körpergewicht um das Körpergewicht eines Menschen handelt.

6. Verwendung nach Anspruch 1,
**dadurch gekennzeichnet, dass**
die Dosis bezogen auf den Anteil an Wirksubstanz kleiner ist als 161 mg / kg Körpergewicht / Tag, vorzugsweise kleiner ist als 50 mg / kg Körpergewicht / Tag, vorzugsweise kleiner ist als 10 mg / kg Körpergewicht / Tag, vorzugsweise kleiner ist als 1 mg / kg Körpergewicht / Tag, vorzugsweise kleiner ist als 0,1 mg / kg Körpergewicht / Tag, wobei es sich bei dem Körpergewicht um das Körpergewicht eines Menschen handelt.

7. Verwendung nach Anspruch 1,
**dadurch gekennzeichnet, dass**
als Dosis bezogen auf den Anteil an Wirksubstanz ein Bereich von 0,01 bis 161 mg / kg Körpergewicht / Tag, vorzugsweise 0,05 bis 120 mg / kg Körpergewicht / Tag, vorzugsweise 0,1 bis 100 mg / kg Körpergewicht / Tag, vorzugsweise 0,5 bis 80 mg / kg Körpergewicht / Tag, vorzugsweise 1 bis 80 mg / kg Körpergewicht / Tag, vorzugsweise 5 bis 80 mg / kg Körpergewicht / Tag, vorzugsweise 10 bis 80 mg / kg Körpergewicht / Tag vorgesehen ist, wobei es sich bei dem Körpergewicht um das Körpergewicht eines Menschen handelt.

8. Verwendung nach einem der vorhergehenden Ansprüche,
**dadurch gekennzeichnet, dass**
eine Zusammensetzung als Wirksubstanz verwendet wird, bei der Xanthohumol mit einem Anteil im Bereich von 0,1 Gew.-% - 99 Gew.-%, vorzugsweise 5 Gew.-% - 99 Gew.-%, vorzugsweise 10 Gew.-% - 99 Gew.-%, vorzugsweise 20 Gew.-% - 99 Gew.-%, vorzugsweise 30 Gew.-% - 99 Gew.-%, vorzugsweise 40 Gew.-% - 99 Gew.-%, vorzugsweise 50 Gew.-% - 99 Gew.-%, vorzugsweise 60 Gew.-% - 99 Gew.-%, vorzugsweise 70 Gew.-% - 99 Gew.-% verwendet wird.

9. Verwendung nach Anspruch 1,
**dadurch gekennzeichnet, dass**
das Xanthohumol in Reinform verwendet wird.

10. Verwendung nach Anspruch 1,
**dadurch gekennzeichnet, dass**
das Xanthohumol in synthetisierter Form verwendet wird.

11. Verwendung nach Anspruch 1,
**dadurch gekennzeichnet, dass**
das Xanthohumol in Verbindung mit mindestens einem weiteren Wirkstoff eingesetzt wird.

12. Verwendung nach Anspruch 1,
**dadurch gekennzeichnet, dass**
das Xanthohumol in Verbindung mit oder auf der Basis von einem Alkali-oder Erdalkalisalz zum Einsatz kommt.

13. Verwendung nach Anspruch 1,
**dadurch gekennzeichnet, dass**
das Xanthohumol als Flüssigkeit, Brei, Emulsion, in Form von Nanopartikeln, als Puder oder als Gel zum Einsatz kommt.

14. Verwendung von Xanthohumol mit der Formel 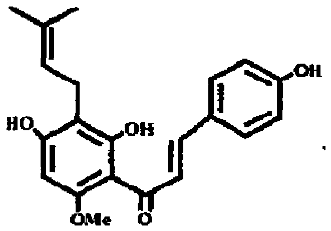 gegebenenfalls in Verbindung mit oder auf der Basis von einem Salz als Wirkstoff für die Herstellung eines Medikaments zur Behandlung der nichtalkoholischen Steatosis-Hepatitis (NASH).

## Claims

1. Use of xanthohumol of the formula 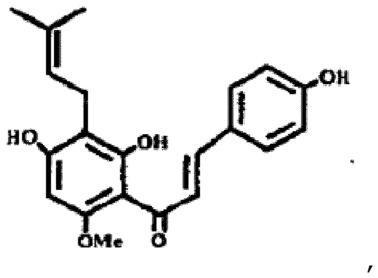 optionally associated with or based on a salt as active compound for preparing a food supplement or medicament for preventing liver damage mediated by adipositas and/or diabetes.

2. Use according to Claim 1,
**characterized in that**
the active compound in question is to be used in a dosage form which ensures that active compound concentrations of a dosage of ≥ 5 µM, in particular ≥ 10 µM, in particular ≥ 20 µM, in particular ≥ 30 µM, in particular ≥ 40 µM, in particular ≥ 50 µM are obtained in the liver.

3. Use according to Claim 1 or 2,
**characterized in that**
the active compound is to be used in a dosage form which ensures that an active compound concentration of at most 100 µM is obtained in the liver.

4. Use according to Claim 1,
**characterized in that**
the xanthohumol is to be used in a dosage form which ensures that active compound concentrations in the following ranges of from 1 to 100 µM, preferably 1 - 25 µM, preferably 1 - 10 µM or 5-100 µM, preferably 10 - 50 µM, preferably 10-25 µM are obtained in the liver.

5. Use according to Claim 1,
**characterized in that**
the dosage, based on the proportion of active substance, is greater than 0.01 mg/kg of bodyweight/day, preferably greater than 0.1 mg/kg of bodyweight/day, preferably greater than 1 mg/kg of bodyweight/day, preferably greater than 10 mg/kg of bodyweight/day, preferably greater than 50 mg/kg of bodyweight/day, preferably greater than 100 mg/kg of bodyweight/day, where the bodyweight is the bodyweight of a human.

6. Use according to Claim 1,
**characterized in that**
the dosage, based on the proportion of active substance, is less than 161 mg/kg of bodyweight/day, preferably less than 50 mg/kg of bodyweight/day, preferably less than 10 mg/kg of bodyweight/day, preferably less than 1 mg/kg of bodyweight/day, preferably less than 0.1 mg/kg of bodyweight/day, where the bodyweight is the bodyweight of a human.

7. Use according to Claim 1,
**characterized in that**
the intended dosage, based on the proportion of active substance, is a range of from 0.01 to 161 mg/kg of bodyweight/day, preferably from 0.05 to 120 mg/kg of bodyweight/day, preferably from 0.1 to 100 mg/kg of bodyweight/day, preferably from 0.5 to 80 mg/kg of bodyweight/day, preferably from 1 to 80 mg/kg of bodyweight/day, preferably from 5 to 80 mg/kg of bodyweight/day, preferably from 10 to 80 mg/kg of bodyweight/day, where the bodyweight is the bodyweight of a human.

8. Use according to any of the preceding claims,
**characterized in that**
as active substance, a composition is used where xanthohumol is used in a proportion in the range of from 0.1% by weight - 99% by weight, preferably 5% by weight - 99% by weight, preferably 10% by weight - 99% by weight, preferably 20% by weight-99% by weight, preferably 30% by weight - 99% by weight, preferably 40% by weight - 99% by weight, preferably 50% by weight - 99% by weight, preferably 60% by weight - 99% by weight, preferably 70% by weight - 99% by weight.

9. Use according to Claim 1,
**characterized in that** the xanthohumol is used in pure form.

10. Use according to Claim 1,
**characterized in that**
the xanthohumol is used in synthesized form.

11. Use according to Claim 1,
**characterized in that**
the xanthohumol is employed in association with at least one further active compound.

12. Use according to Claim 1,
**characterized in that**
the xanthohumol is employed associated with or based on an alkali metal salt or alkaline earth metal salt.

13. Use according to Claim 1,
**characterized in that**
the xanthohumol is employed as a liquid, a paste, an emulsion, in the form of nanoparticles, as a powder or as a gel.

14. Use of xanthohumol of the formula 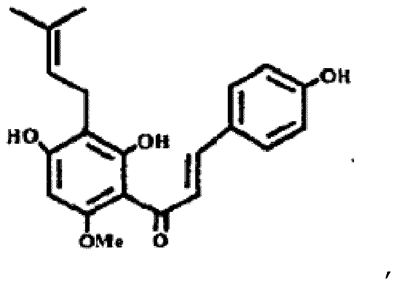 optionally associated with or based on a salt as active compound for preparing a medicament for treating non-alcoholic steatohepatitis (NASH).

## Revendications

1. Utilisation de xanthohumol de formule 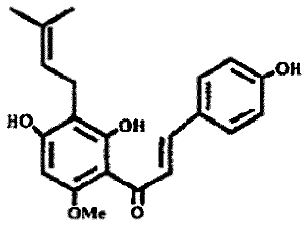 éventuellement conjointement avec ou à base d'un sel en tant que principe actif pour la fabrication d'un complément alimentaire ou d'un médicament pour la prévention des lésions du foie médiées par l'adiposité et/ou le diabète.

2. Utilisation selon la revendication 1, **caractérisée en ce que** le principe actif respectif doit être utilisé en une forme pharmaceutique telle que des concentrations de principe actif dans une dose ≥ 5 µM, notamment ≥ 10 µM, notamment ≥ 20 µM, notamment ≥ 30 µM, notamment ≥ 40 µM, notamment ≥ 50 µM, s'ajustent dans le foie.

3. Utilisation selon la revendication 1 ou 2, **caractérisée en ce que** le principe actif doit être utilisé en une forme pharmaceutique telle qu'une concentration de principe actif d'au plus 100 µM s'ajuste dans le foie.

4. Utilisation selon la revendication 1, **caractérisée en ce que** le xanthohumol doit être utilisé en une forme pharmaceutique telle que des concentrations de principe actif dans les plages suivantes s'ajustent dans le foie : 1 à 100 µM, de préférence 1 à 25 µM, de préférence 1 à 10 µM ou 5 à 100 µM, de préférence 10 à 50 µM, de préférence 10 à 25 µM.

5. Utilisation selon la revendication 1, **caractérisée en ce que** la dose par rapport à la proportion de substance active est supérieure à 0,01 mg/kg de poids corporel/jour, de préférence supérieure à 0,1 mg/kg de poids corporel/jour, de préférence supérieure à 1 mg/kg de poids corporel/jour, de préférence supérieure à 10 mg/kg de poids corporel/jour, de préférence supérieure à 50 mg/kg de poids corporel/jour, de préférence supérieure à 100 mg/kg de poids corporel/jour, le poids corporel étant le poids corporel d'un homme.

6. Utilisation selon la revendication 1, **caractérisée en ce que** la dose par rapport à la proportion de substance active est inférieure à 161 mg/kg de poids corporel/jour, de préférence inférieure à 50 mg/kg de poids corporel/jour, de préférence inférieure à 10 mg/kg de poids corporel/jour, de préférence inférieure à 1 mg/kg de poids corporel/jour, de préférence inférieure à 0,1 mg/kg de poids corporel/jour, le poids corporel étant le poids corporel d'un homme.

7. Utilisation selon la revendication 1, **caractérisée en ce qu'**en tant que dose par rapport à la proportion de substance active, une plage allant de 0,01 à 161 mg/kg de poids corporel/jour, de préférence de 0,05 à 120 mg/kg de poids corporel/jour, de préférence de 0,1 à 100 mg/kg de poids corporel/jour, de préférence de 0,5 à 80 mg/kg de poids corporel/jour, de préférence de 1 à 80 mg/kg de poids corporel/jour, de préférence de 5 à 80 mg/kg de poids corporel/jour, de préférence de 10 à 80 mg/kg de poids corporel/jour, est prévue, le poids corporel étant le poids corporel d'un homme.

8. Utilisation selon l'une quelconque des revendications précédentes, **caractérisée en ce qu'**une composition est utilisée en tant que substance active, dans laquelle le xanthohumol est utilisé en une proportion dans la plage allant de 0,1 % en poids à 99 % en poids, de préférence de 5 % en poids à 99 % en poids, de préférence de 10 % en poids à 99 % en poids, de préférence de 20 % en poids à 99 % en poids, de préférence de 30 % en poids à 99 % en poids, de préférence de 40 % en poids à 99 % en poids, de préférence de 50 % en poids à 99 % en poids, de préférence de 60 % en poids à 99 % en poids, de préférence de 70 % en poids à 99 % en poids.

9. Utilisation selon la revendication 1, **caractérisée en ce que** le xanthohumol est utilisé sous forme pure.

10. Utilisation selon la revendication 1, **caractérisée en ce que** le xanthohumol est utilisé sous forme synthétisée.

11. Utilisation selon la revendication 1, **caractérisée en ce que** le xanthohumol est utilisé conjointement avec au moins un autre principe actif.

12. Utilisation selon la revendication 1, **caractérisée en ce que** le xanthohumol est utilisé conjointement avec ou à base d'un sel alcalin ou alcalino-terreux.

13. Utilisation selon la revendication 1, **caractérisée en ce que** le xanthohumol est utilisé sous la forme d'un liquide, d'une bouillie, d'une émulsion, sous la forme de nanoparticules, sous la forme d'une poudre ou sous la forme d'un gel.

14. Utilisation de xanthohumol de formule 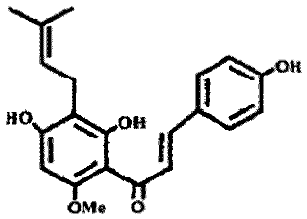 éventuellement conjointement avec ou à base d'un sel en tant que principe actif pour la fabrication d'un médicament pour le traitement de l'hépatite stéatosique non alcoolique (NASH).
